(19) Europäisches Patentamt
European Patent Office
Office européen des brevets

(11) Publication number: **0 428 106 A1**

(12) # EUROPEAN PATENT APPLICATION

(21) Application number: **90121633.3**

(51) Int. Cl.5: **C07D 413/04**, A61K 31/42

(22) Date of filing: **12.11.90**

(30) Priority: **13.11.89 US 435314**

(43) Date of publication of application:
**22.05.91 Bulletin 91/21**

(84) Designated Contracting States:
**AT BE CH DE DK ES FR GB GR IT LI LU NL SE**

(71) Applicant: **MERRELL DOW PHARMACEUTICALS INC.**
**2110 East Galbraith Road**
**Cincinnati Ohio 45215(US)**

(72) Inventor: **Cheng, Hsien C.**
**12079 Cantrell Drive**
**Cincinnati, Ohio 45246(US)**
Inventor: **Dage, Richard C.**
**7825 Shadowhill Way**
**Cincinnati, Ohio 45242(US)**
Inventor: **Jones, Winton D.**
**1464 Longacre Drive**
**Cincinnati, Ohio 45240(US)**
Inventor: **Robinson, Phillip J.**
**41 Victoria Parade**
**Fitzroy, Victoria 3085(AU)**

(74) Representative: **Vossius & Partner**
**Siebertstrasse 4 P.O. Box 86 07 67**
**W-8000 München 86(DE)**

(54) **Novel pyridyloxazole 2-ones useful as proteinkinase C inhibitors.**

(57) This invention relates to novel pyridyloxazole-2-ones which are useful as protein kinase C inhibitors, effective in the treatment of hypertension and asthma.

EP 0 428 106 A1

## NOVEL PYRIDYLOXAZOLE-2-ONES USEFUL AS PROTEINKINASE C INHIBITORS

This invention relates to certain pyridinyloxazole-2-ones of the formula

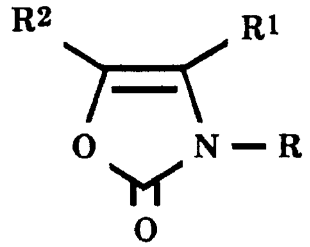

(1)

wherein

R and $R^1$ are each independently selected from the group consisting of hydrogen, $C_1$-$C_6$ alkyl, and phenyl or $C_1$-$C_3$ alkylphenyl wherein the phenyl ring is optionally substituted with one, two or three of the substituents selected from the group consisting of fluorine, chlorine, bromine, $C_1$-$C_4$ alkyl, and $C_1$-$C_4$ alkoxy; and

$R^2$ is a 2-, 3-, or 4-pyridyl group wherein the pyridyl group is optionally substituted with one or two substituents selected from the group consisting of fluorine, chlorine, bromine, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, cyano, carboxy, carb($C_1$-$C_5$)alkoxy, carbamido, ($C_1$-$C_5$)alkanoylamino, imidazolyl, nitro and trifluoromethyl or wherein the pyridyl group is optionally substituted with a phenyl group which is optionally substituted with one, two or three of the substituents selected from the group consisting of fluorine, chlorine, bromine, $C_1$-$C_4$ alkyl, and $C_1$-$C_4$ alkoxy;

and to the pharmaceutically acceptable salts thereof.

This invention also concerns the use of the compounds of Formula I as protein kinase C inhibitors effective as vasodilators in the treatment of hypertension and as bronchodilators in the treatment of asthma. This invention also concerns a process for making certain intermediate ketone compounds which are useful in the synthesis of the compounds of Formula I.

As used herein, the terms "$C_1$-$C_3$ alkyl", "$C_1$-$C_4$ alkyl" and "$C_1$-$C_6$ alkyl" mean straight or branched chain alkyl groups having from one to three, from one to four, or from one to six carbon atoms respectively, and include such groups as methyl, ethyl, n-propyl, isopropyl, n-butyl, isobutyl, sec-butyl, tert-butyl, and the like, as well as vinyl, allyl, propynyl, butenyl, butadienyl, isopropenyl, and the like. The term "$C_1$-$C_4$ alkoxy" means alkoxy groups having from one to four carbon atoms, and includes such groups as methoxy, ethoxy, n-propoxy, isopropoxy, n-butoxy, isobutoxy, sec-butoxy, tert-butoxy, and the like. When R or $R^1$ is "optionally substituted phenyl or $C_1$-$C_3$ alkylphenyl", the one, two or three substituent(s) can be located at any available position on the phenyl ring. When $R^2$ is 2-, 3-, or 4-pyridinyl the optional substituent(s) can be located at any available position(s) on the pyridinyl ring.

Illustrative examples of the compounds of this invention include compounds of Formula I wherein the R groups are designated as follows:

| R | $R^1$ | $R^2$ |
|---|---|---|
| hydrogen | hydrogen | 2-,3-, or 4-pyridinyl |
| ethyl | hydrogen | 2-,3-, or 4-pyridinyl |
| propyl | hydrogen | 2-,3-, or 4-pyridinyl |
| methyl | benzyl | 2-,3-, or 4-pyridinyl |
| phenethyl | hydrogen | 2-,3-, or 4-pyridinyl |
| phenyl | hydrogen | 2-,3-, or 4-pyridinyl |
| propyl | hydrogen | 2-, 3- or 4-(3,4-dimethyl)pyridinyl |
| propyl | hydrogen | 2-,3-,or4-(3-phenyl)pyridinyl |
| 4-methoxyphenethyl | hydrogen | 2, 3- or 4-pyridinyl |
| 4-methoxyphenyl | hydrogen | 2, 3- or 4-pyridinyl |
| benzyl | benzyl | 2-, 3- or 4-(2-ethoxy)pyridinyl |
| phenyl | benzyl | 2-, 3- or 4-(2-ethoxy)pyridinyl |
| butyl | hydrogen | 2-, 3- or 4-pyridinyl |
| 3,5-(dichloro)phenylpropyl | methyl | 2-, 3- or 4-pyridinyl |
| 3,5-(dichloro)phenyl | methyl | 2-, 3- or 4-pyridinyl |
| propyl | methyl | 2-, 3- or 4-pyridinyl |
| 3,5-dimethoxybenzyl | ethyl | 2-, 3- or 4-pyridinyl |
| 3,5-dimethoxyphenyl | ethyl | 2-, 3- or 4-pyridinyl |
| methyl | propyl | 2-, 3- or 4-(3-ethoxy-4-methyl)pyridinyl |
| methyl | propyl | 2-,3-,or4-(3-phenyl)pyridinyl |
| butyl | butyl | 2-, 3-, or 4-pyridinyl |
| hydrogen | phenethyl | 2-, 3- or 4-(4-trifluoromethyl)pyridinyl |
| hydrogen | phenethyl | 2-,3-, or 4-(4-phenyl)pyridinyl |
| methyl | 4-methoxy-phenethyl | 2-, 3- or 4-pyridinyl |

As is true for most classes of therapeutically effective compounds, certain subclasses and certain species are especially effective and are preferred over others. In this instance, those compounds of Formula I wherein $R^2$ is optionally substituted 2-, 3-, or 4-pyridinyl are preferred. Also preferred are compounds wherein R is hydrogen or a $C_1$-$C_6$ alkyl. Most preferred are the compounds wherein $R^2$ is an unsubstituted 2-, 3-, or 4-pyridinyl group, R is propyl and $R^1$ is hydrogen. The most preferred compound of this invention is 4-propyl-5-(4-pyridinyl)-2(3H)-oxazolone.

The preparation of the 2-, 3-, or 4-pyridinyloxazole-2-ones of this invention is known in the art. See for example, United States Patent Number 4,698,353. The preparation of those compounds not specifically taught in the art can be readily accomplished by the skilled artisan.

In essence, the compounds of this invention can be prepared by reacting a compound of formula 2

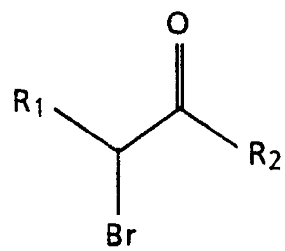

(2)

wherein $R_1$ and $R_2$ are as defined above with a cyanate or thiocyanate salt to form the corresponding isocyanate or isothiocyanate of formula 3

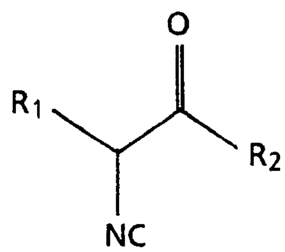

(3)

wherein $R_1$ and $R_2$ are as defined above. The isocyanate compound when heated, typically as a melt at from 90° to 110° C, without solvent condenses to form a compound of formula 1.

Another procedure involves cyclizing a hydroxyketone of structure 4

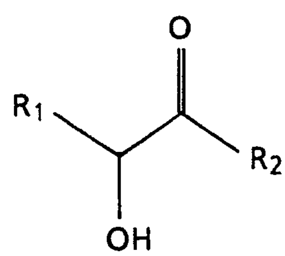

(4)

wherein $R_1$ and $R_2$ are as defined above by reaction with a cyanate or salt in the presence of an acid.

The bromo-ketones of formula 2 are either known in the art or can be readily prepared by standard techniques. For example the des-bromo analog of a structure 2 compound can be treated with bromine. Where the group adjacent to the carbon to be brominated is a hydrogen or a ($C_1$-$C_5$) alkyl group, a radical initiator can be used to promote the bromination. Suitable initiators include iron metal and N-bromosuccinimide. The bromination can also be accomplished by the addition of concentrated hydrobromic acid, typically 48% aqueous hydrobromic acid, to a solution containing the des-bromo compound. The structure 4 hydroxyketones can also be readily prepared in any suitable manner. For example, a structure 2 bromoketone can be allowed to react with an acetate salt, preferably potassium acetate, to form the corresponding acetyloxyketone which upon treatment with an acid, such as hydrochloric acid, yields the desired structure 4 compound.

The compounds wherein R is $C_1$-$C_6$ alkyl or optionally substituted phenyl or $C_1$-$C_3$ alkylphenyl are produced by subsequent reaction of the compound of Formula 1 wherein R is hydrogen with sodium hydride and the appropriate alkyl iodide or phenylalkyl iodide in tetrahydrofuran according to procedures well known in the art.

The compounds of this invention are useful both in the free base form and as salts. The expression "pharmaceutically-acceptable salt" means any organic or inorganic addition salt of the base compounds of Formula I which are relatively non-toxic and innocuous to a patient at concentrations consistent with effective activity so that the side effects ascribable to the salt do not vitiate the beneficial effects of the base compounds of Formula I. These salts are included within the scope of this invention. Such salts include alkali metal salts, such as sodium and potassium salts and alkaline earth metal salts, such as calcium and

magnesium salts; and the like. Also salts with organic and inorganic acids can be prepared, such as, for example, those formed with the following acids: hydrochloric, hydrobromic, sulfonic, sulfuric, phosphoric, nitric, ascorbic, methanesulfonic, acetic, propionic, tartaric, citric, lactic, malic, mandelic, cinnamic, palmitic, itaconic, fumaric, benzenesulfonic and toluenesulfonic. The non-toxic, physiologically acceptable salts are preferred, although other salts are also useful, for example, in isolating or purifying the product.

The salts can be formed by conventional means such as by reacting the free acid or free base forms of the product with one or more equivalents of the appropriate base or acid in a solvent or medium in which the salt is insoluble, or in a solvent such as water which is then removed *in vacuo* or by freeze-drying, or by exchanging the cations of an existing salt for another cation on a suitable ion exchange resin.

The compounds of Formula I are inhibitors of Protein Kinase C (PKC) and can be used be lower blood pressure and are bronchodilators. As such, the compounds of this invention are useful in the treatment of hypertension and as agents in the treatment of, for example, asthma.

A patient, for the purpose of this invention, is a mammal, including a human, in need of treatment for a particular condition, injury or disease. The amount of active ingredient (i.e., a compound of Formula I) to be administered to a patient for the treatment of hypertension can vary widely according to such considerations as the particular compound and dosage unit employed, the period of treatment, the age and sex of the patient treated, and the extent of the hypertension treated.

The total amount of active ingredient to be administered intravenously will generally range from about 0.1 mg/kg to 30 mg/kg and preferably from 1.0 mg/kg to 10.0 mg/kg. A unit dosage may contain from 5 mg to 525 mg of active ingredient, and can be taken one or more times per day. For example, a 50 kg patient may be administered 50 mg - 700 mg active ingredient four times a day for a total dose of 200 mg - 2800 mg per day.

The total amount of active ingredient to be administered orally will generally range from 0.1 mg/kg to 100 mg/kg, and preferably from 1.0 mg/kg to 50 mg/kg. A unit dosage may contain from 5 mg to 1000 mg of active ingredient, and can be taken one or more times per day. For example, a 50 kg patient may be administered 50 mg - 2500 mg of active ingredient four times a day for a total of 200 mg - 10,000 mg per day.

The compounds of this invention can be administered as the sole anti-hypertensive agent or in combination with other anti-hypertensive agents and/or diuretic agents and/or calcium entry blocking agents. For example, the compounds of this invention can be given in combination with such compounds as benzthiazide, clonidine, deserpidine, furosemide, hydralazine hydrochloride, indacrinone and variable ratios of its enantiomers, prazosin, propranolol, reserpine, and the like, as well as admixtures and combinations thereof.

Typically, the individual daily dosages for these combinations can range from about one fifth of the minimally recommended clinical dosages to the maximum recommended levels for the entities when they are given singly.

When the compounds of this invention are administered as a bronchodilatory agents in the treatment of, for example, asthma, the amount of active ingredient to be administered can vary widely according to such considerations as the particular compound and dosage unit employed, the period of treatment, the age and sex of the patient treated, and the extent of the condition treated. The total amount of active ingredient to be administered parenterally or by inhalation will generally range from about 0.1 mg/kg to 30.0 mg/kg and preferably from 1 mg/kg to 10 mg/kg. A unit dosage may contain from 5 mg to 525 mg of active ingredient, and can be taken one or more times per day. For example, a 50 kg patient may be administered 50 mg to 700 mg active ingredient, four times a day for a total dose of 200 mg-2800 mg per day.

The compounds of this invention can be utilized to achieve the desired pharmacological effect by administration to a patient in need thereof in an appropriately formulated pharmaceutical composition. Therefore, the present invention includes pharmaceutical compositions which are comprised of a pharmaceutically acceptable carrier and a pharmaceutically effective amount of a compound of Formula I. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated. The compounds of Formula I can be administered with a pharmaceutically acceptable carrier using conventional dosage unit forms orally, parenternally, topically, as an aerosol, or the like.

For oral administration the compounds can be formulated into solid or liquid preparations such as capsules, pills, tablets, troches, lozenges, melts, powders, solutions, suspensions, or emulsions and may be prepared according to methods known to the art for the manufacture of pharmaceutical compositions. The solid unit dosage forms can be a capsule which can be of the ordinary hard- or soft-shelled gelatin type

containing, for example, surfactants, lubricants, and inert fillers such as lactose, sucrose, calcium phosphate, and cornstarch.

In another embodiment, the compounds of this invention may be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quaternary ammonium salts, as well as mixtures.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethyl-cellulose, methylcellulose, hydroxypropylmethyl-cellulose, sodium alginate, polyvinylpyrrolidone, gum tragacanth and gum acacia; dispersing or wetting agents which may be a naturally-occurring phosphatide such as lecithin, a condensation product of an alkylene oxide with a fatty acid, for example, polyoxyethylene stearate, a condensation product of ethylene oxide with a long chain aliphatic alcohol, for example, heptadecaethyleneoxycetanol, a condensation product of ethylene oxide with a partial ester derived from a fatty acid and a hexitol such as polyoxyethylene sorbitol monooleate, or a condensation product of an ethylene oxide with a partial ester derived from a fatty acid and a hexitol anhydride, for example, polyoxyethylene sorbitan monooleate.

The suspensions may also contain one or more preservatives, for example, ethyl or n-propyl p-hydroxybenzoate; one or more coloring agents; one or more flavoring agents; and one or more sweetening agents such as sucrose or saccharin.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin or cetyl alcohol.

The sterile injectable preparation may also be a sterile injectable solution or suspension in a non-toxic parenterally-acceptable diluent or solvent. Diluents and solvents that may be employed are, for example, water, Ringer's solution, and isotonic sodium chloride solution. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are, for example, cocoa butter and polyethylene glycol.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring, and coloring agents described above, may also be present.

The compounds of this invention may be formulated as solutions, suspensions, emulsions, powders, and semisolid preparations administered as an aerosol preparation by means of a pressurized aerosol container together with a gaseous or liquefied propellant such as, for example, dichlorodifluoromethane, dichlorodifluoromethane with dichlorodifluoroethane, carbon dioxide, nitrogen, propane, or the like, with the usual adjuvants such as co-solvents and wetting agents, as may be necessary or desirable. The compounds may also be administered in a non-pressurized form such as in a nebulizer or atomizer. The aerosols are intended for administration as fine, solid particles or as liquid mists via the respiratory system, and the particle size of aerosol preparations intended for administration to the lungs should be below 50 micrometers, in most instances.

The pharmaceutical compositions of the invention may also be in the form of oil-in-water emulsions. The oily phase may be a vegetable oil such as liquid paraffin or a mixture of vegetable oils. Suitable emulsifying agents may be (1) naturally-occurring gums such as gum acacia and gum tragacanth, (2) naturally-occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents such as, for example, glycerol, propylene glycol, sorbitol or sucrose. Such formulations may also contain a demulcent, and preservative and flavoring and coloring agents.

The compositions of the invention can also contain other conventional pharmaceutically-acceptable compounding ingredients, generally referred to as carriers or diluents, as necessary or desired. Any of the compositions of this invention may be preserved by the addition of an antioxidant such as ascorbic acid or by other suitable preservatives. Conventional procedures for preparing such compositions in appropriate dosage forms can be utilized.

The following specific examples are presented to illustrate the synthesis of the compounds of this invention, but they should not be construed as limiting the scope of this invention in any way.

## EXAMPLE 1

### 4-Ethyl-5-Pyridin-4-yl-2(3H)-Ozazolone

1-Hydroxy-2-(4-pyridyl)butan-2-one (26.4 g, 0.16 mol) was dissolved in 350 ml of 2N HCl. Potassium cyanate (38.9 g, 0.48 mol) was added portionwise to this solution over a period of one hour with stirring. After the addition was complete, concentrated hydrochloric acid was added until the pH of the solution was one. After an additional hour the reaction mixture was made basic by addition of sodium bicarbonate solution and the resulting mixture was stirred overnight. The resulting solid precipitate was collected and recrystallized twice from 50% aqueous ethanol to yield the title compound (14.4 g, 47% of theoretical yield), m.p. 287°-289° C. (dec.).

Using the procedure above but using 1-(hydroxy)-1-(4-pyridyl)pentan-2-one or 1-(hydroxy)-1-(4-pyridyl)-propan-2-one instead of 1-hydroxy-1-(4-pyridyl)butan-2-one results in 4-propyl-5-pyridin-4-2(3H)-oxazolone, m.p. 257° -259° C (dec.) or 4-methyl-5-pyridin-4-yl-2(3H)-oxazolone, m.p. >310° C.

## EXAMPLE 2

### 4-Ethyl-5-(2-pyridyl)-2(3H)-oxazolone

Potassium cyanate (35.4 g, 0.44 mol) was added to a solution of 2-hydroxy-1-(2-pyridyl)butan-1-one (31 g, 0.15 mol) in 250 ml of 2N HCl diluted with 300 ml of water. After 1 hour the acidity was adjusted (pH = 1) with concentrated hydrochloric acid and then allowed to stir overnight. The mixture was made basic by addition of aqueous sodium bicarbonate. The resulting gummy precipitate was chromatographed on silca gel and recrystallized twice from 50% aqueous ethanol to give the title compound, m.p. 196° -197° C. (dec.).

In a manner similar to that set forth in Examples 1 and 2, the compounds 4-phenyl-5-pyridin-4-yl-2(3H)-oxazolone (mp > 300° C) and 4-propyl-5-(2-phenylpyridin-4-yl)-2(3H)-oxazolone(mp 202-204° C) were prepared.

| EXAMPLE 3 | |
|---|---|
| A tablet is prepared from | |
| 4-propyl-5-pyridin-4-yl-2(3H)-oxazolone | 250 mg |
| starch | 40 mg |
| talc | 10 mg |
| magnesium stearate | 10 mg |

| EXAMPLE 4 | |
|---|---|
| A capsule is prepared from | |
| 4-ethyl-5-pyridin-4-yl-2(3H)-oxazolone | 400 mg |
| talc | 40 mg |
| sodium carboxymethylcellulose | 40 mg |
| starch | 120 mg |

| EXAMPLE 5 | |
|---|---|
| A tablet is prepared from | |
| 4-Methyl-5-(3-pyridinyl-1-(3H)-oxazolone | 250 mg |
| Starch | 40 mg |
| Talc | 10 mg |
| Magnesium | 10 mg |

| EXAMPLE 6 | |
|---|---|
| A capsule is prepared from | |
| 4-phenyl-5-(2-pyridinyl)1-(3H)-oxazolone | 400 mg |
| Talc | 40 mg |
| Sodium Carboxymethyl cellulose | 40 mg |
| Starch | 120 mg |

The compounds of Formula I may also be utilized, in free base form or in compositions, in research and diagnostics, or as analytical references or standards, and the like.

Therefore, the present invention includes compositions which are comprised of an inert carrier and an effective amount of a compound of Formula I, or a salt thereof. An inert carrier is any material which does not interreact with the compound to be carried and which lends support, means of conveyance bulk, traceable material, and the like to the compound to be carried. An effective amount of compound is that amount which produces a result or exerts an influence on the particular procedure being performed.

It should be apparent to one of ordinary skill in the art that changes and modifications can be made to this invention without departing from the spirit or scope of the invention as it is set forth herein.

## Claims

1. Use of compounds of the formula

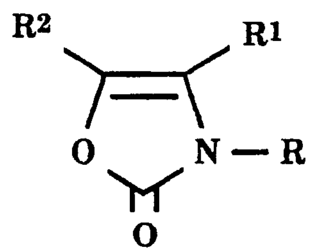

(I)

wherein

R and $R^1$ are each independently selected from the group consisting of hydrogen, $C_1$-$C_6$ alkyl, and phenyl or $C_1$-$C_3$ alkylphenyl wherein the phenyl ring is optionally substituted with one, two or three of the substituents selected from the group consisting of fluorine, chlorine, bromine, $C_1$-$C_4$ alkyl, and $C_1$-$C_4$ alkoxy; and

$R^2$ is a 2-, 3-, or 4-pyridyl group wherein the pyridyl group is optionally substituted with one or two substituents selected from the group consisting of fluorine, chlorine, bromine, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, cyano, carboxy, carb($C_1$-$C_5$)alkoxy, carbamido, ($C_1$-$C_5$)alkanoylamino, imidazolyl, nitro and trifluoromethyl or wherein the pyridyl group is optionally substituted with a phenyl group which is optionally substituted with one, two or three of the substituents selected from the group consisting of fluorine, chlorine, bromine, $C_1$-$C_4$ alkyl, and $C_1$-$C_4$ alkoxy;

or the pharmaceutically-acceptable salts thereof for the production of a medicament for the inhibition of protein kinase C.

2. Use according to claim 1 of compounds of the formula (I) or the pharmaceutically-acceptable salts thereof for the production of a medicament for the treatment of hypertension.

3. Use according to claim 1 of compounds of the formula (I) or the pharmaceutically-acceptable salts thereof for the production of a medicament for the treatment of asthma.

4. The use according to any one of claims 1 to 3 wherein in the compound of the formula I $R^2$ is an optionallly substituted 2-, 3-, or 4-pyridyl group.

5. The use according to any one of claims 1 to 4 wherein in the compound of the formula I R and $R^1$ are each independently selected from the group consisting of hydrogen or $C_1$-$C_6$ alkyl.

6. The use according to claim 5 wherein in the compound of the formula I R is $C_1$-$C_6$ alkyl and $R^1$ is hydrogen.

7. The use according to claim 6 wherein in the compound of the formula I $R^2$ is an unsubstituted 2-, 3-, or 4-pyridyl group.
8. The use according to claim 7 wherein in the compound of the formula I $R^2$ is 4-pyridyl, R is propyl, and $R^1$ is hydrogen.
9. The use according to any one of claims 1 to 3 wherein in the compound of the formula I R is hydrogen, $R^1$ is phenyl, and $R^2$ is 4-pyridyl.
10. The use according to any one of claims 1 to 3 wherein in the compound of the formula I R is hydrogen, $R^1$ is propyl, and $R^2$ is 2-phenylpyridin-4-yl.

European Patent Office

# EUROPEAN SEARCH REPORT

Application Number

EP 90121633.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP - A1 - 0 222 358 (MERRELL DOW) * Claims 1-11 * -- | 1 | C 07 D 413/04<br>A 61 K 31/42 |
| A | EP - A2 - 0 323 171 (KYOWA HAKKO) * Abstract * -- | 1 | |
| A | DE - A1 - 2 935 902 (NATTERMANN) * Formula II * -- | 1 | |
| A | CHEMICAL ABSTRACTS, vol. 111, no. 3, July 17, 1989, Columbus, Ohio, USA ISHAQ, KHALID S. et al. "Synthesis and biological evaluation of ether-linked derivatives of phosphatidy-linositol" page 20, column 1, abstract-No. 17 222g & Pharm. Res. 1989, 6(3), 216-24 -- | 1 | TECHNICAL FIELDS SEARCHED (Int. Cl.5)<br>C 07 D 413/00 |
| | CHEMICAL ABSTRACTS, vol. 106, no. 7, February 16, 1987, Columbus, Ohio, USA UFF, BARRIE C. et al. "Studies with Reissert compounds. Part 16. Some reactions of N-alkoxycarbonyl Reissert compounds with heterocumulenes: formation of the imidazo(5,1-a)isoquinoline and imidazo(5,1-a)-phthalazine systems and related chemistry" page 635, column 1, abstract -No. 50 112r | 1 | |

The present search report has been drawn up for all claims

| Place of search | Date of completion of the search | Examiner |
|---|---|---|
| VIENNA | 02-01-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-
EP 90121633.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | & J. Chem. Res., Synop. 1986, (6), 206-7<br>---- | | |
| | | | TECHNICAL FIELDS SEARCHED (Int. Cl.5) |

The present search report has been drawn up for all claims

| Place of search | Date of completion of the search | Examiner |
|---|---|---|
| VIENNA | 02-01-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)